# EUROPEAN PATENT APPLICATION

(11) **EP 2 311 977 A1**
(43) Date of publication of application: **20.04.2011**
(21) Application number: 09305984.8
(22) Date of filing: 15.10.2009
(51) Int. Cl.: C12Q 1/68

(54) **Method for predicting responsiveness of a patient to a chemoradiation treatment**

(71) Applicant: INSERM (Institut National de la Santé et de la Recherche Medicale), 75013 Paris (FR)
(72) Inventor: The designation of the inventor has not yet been filed
(74) Representative: Hirsch, Denise Marie

(57) **Abstract**

The present invention relates to an *in vitro* method for predicting the responsiveness of a patient afflicted with a rectal cancer to a chemoradiation treatment, said method comprising the step of determining in a biological sample obtained from said patient the genotype of the following single nucleotide polymorphisms (SNP):
- rs744154 G>C in the ERCC4 gene,
- rs4880 T>C in the SOD2 gene,
- rs1800925 C>T in the IL-13 gene, or
- rs1800734 G>A in the MLH1 gene.

## Description

### FIELD OF THE INVENTION

The present invention relates to a method for predicting the response of a patient to a chemoradiation treatment.

### BACKGROUND OF THE INVENTION

The association of chemotherapy and radiotherapy, named chemoradiation or radiochemotherapy, is now often used for the treatment of numerous cancers and is recently part of therapeutic standards for different carcinomas. Particularly, such treatments are currently used for the management of rectal cancers.

Studies show that this kind of treatments permits local control and survival improvement Furthermore, in locally advanced stages of cancer, surgery is increasingly supported by a preoperative radiochemotherapy, which can also induce tumor regression and may allow subsequent radical surgery in a primarily nonresectable tumor.

However, means of predicting an individual patient response to such radiochemotherapy are still needed. Identification of predictive markers of cancer response to chemoradiation treatment is of clinical importance, firstly because patients with a priori resistant tumor could be selected for alternative or more intensive treatment regimens aimed at improving their response, but also because of the elevated costs and the important side effects of these treatments.

Many translational studies have sought to identify predictive factors for tumor response after neoadjuvant chemoradiation for rectal cancer (Smith F.M. et al., 2006; Kuremsky J.G. et al., 2009). These studies focused primarily on the immunohistochemical analysis of a small panel of proteins involved in apoptosis (p53, BCL2), cell cycle arrest (p21), proliferation (Ki67), neoangiogenesis (VEGF), DNA repair (MLH1, MSH2) or 5-fluorouracil metabolism (TYMS). So far, conflicting results have been obtained, maybe due to the heterogeneity of the tumoral expression of the studied markers (Spindler KG et al, 2007) or to the lack of standardization of immunohistochemistry (Grube D, 2004).

Single nucleotide polymorphisms (SNPs) are the most common type of germ-line genetic variation, and, millions of SNPs are now described (The International HapMap Consortium). Gene SNPs may alter the function or the expression of the corresponding proteins, and several pharmacogenetic studies have shown that these polymorphisms may contribute to inter-individual variability in efficacy of various anti-cancer treatments (Ulrich C.M. et al., 2003; Robert J. et al., 2005). Furthermore, SNP identification corresponds to a non invasive blood analysis. For these reasons, SNP may be good predictive markers of a response to treatment.

### SUMMARY OF THE INVENTION

The present invention relates to an *in vitro* method for predicting the responsiveness of a patient afflicted with a rectal cancer to a chemoradiation treatment, said method comprising the step of determining in a biological sample obtained from said patient the genotype of the following single nucleotide polymorphisms (SNP):
- rs744154 G>C in the ERCC4 gene,
- rs4880 T>C in the SOD2 gene,
- rs1800925 C>T in the IL-13 gene, or
- rs1800734 G>A in the MLH1 gene.

### DETAILED DESCRIPTION OF THE INVENTION

The inventors studied genetic markers potentially implicated in the response of a patient to a chemoradiation treatment and identified four single nucleotide polymorphisms that are independent predictors of the sensitivity or resistance of the patient to chemoradiation treatment.

Particularly, combinations of particular genotypes for each of these SNPs are predictive of responsiveness or non-responsiveness to the treatment.

### Definitions

Throughout the specification, several terms are employed and are defined in the following paragraphs.

A "*coding sequence*" or a sequence "*encoding*" an expression product, such as a RNA, polypeptide, protein, or enzyme, is a nucleotide sequence that, when expressed, results in the production of that RNA, polypeptide, protein, or enzyme, i.e., the nucleotide sequence encodes an amino acid sequence for that polypeptide, protein or enzyme. A coding sequence for a protein may include a start codon (usually ATG) and a stop codon.

The term *"SNP"* or *"Single Nucleotide Polymorphism"* has its general meaning in the art and refers to a single nucleotide variation in a genetic sequence that occurs at appreciable frequency in the population. The single nucleotide variation can be a substitution but also an addition or a deletion. There are millions of SNPs in the human genome. Most commonly, these variations are found in the DNA between genes. When SNPs occur within a gene or in a regulatory region near a gene, they may play a more direct role in disease by affecting the gene function.

According to the present invention, the term *"genotype"* means the 5' to 3' nucleotide sequence found at a set of one or more polymorphic sites in a locus on both chromosomes from a single individual.

Accordingly, for example, a genotype G/G for a given SNP means that in both copies of the gene of a patient, a G is present at the locus of said polymorphism (when the sequence is read in the 5' to 3' sense), whereas a genotype C/G for said SNP mean that in a first copy of the gene of said patient, a C is present and in the second copy, a G is present.

The term "*ERCC4*" has its general meaning in the art and refers to the *"excision repair cross-complementing rodent repair deficiency, complementation group 4",* a gene encoding a protein implicated in the nucleotide excision repair (NER) pathway. The term may include naturally occurring ERCC4 and variants and modified forms thereof. The ERCC4 can be from any source, but typically is a mammalian (e.g., human and non-human primate) ERCC4, particularly a human ERCC4. An exemplary human native ERCC4 amino acid sequence is provided in GenPept database under accession number NP_005227 and an exemplary human native nucleotide sequence encoding for ERCC4 is provided in GenBank database under accession number NM_005236.

The SNP rs744154 is well known in the art and is notably described in the NCBI database dbSNP. It consists in a variation of a guanine in a cytosine and is located in the first intron of the ERCC4 gene sequence, at the position IVS 1-807 according to the numbering system described in den Dunnen T. et al, 2001 (written IVSI-807G>C in related nomenclature).

The term "*SOD2*" has its general meaning in the art and refers to a gene encoding the mitochondrial "*superoxide dismutase* 2", or SOD2 protein, implicated in the detoxification of reactive oxygen species. The term may include naturally occurring SOD2 and variants and modified forms thereof. The SOD2 can be from any source, but typically is a mammalian (e.g., human and non-human primate) SOD2, particularly a human SOD2. An exemplary human native SOD2 amino acid sequence is provided in GenPept database under accession number NP_000627 and an exemplary human native nucleotide sequence encoding for SOD2 is provided in GenBank database under accession number NM_000636.

The SNP rs4880 is well known in the art and is notably described in the NCBI database dbSNP. It consists in a variation of a thymine in a cytosine and is located in the second exon of the SOD2 gene sequence, at the position Ex2+24 according to the numbering system described in den Dunnen T. et al, 2001 (written Ex2+24T>C in related nomenclature).

The term *"IL13"* or *"interleukin* 13" has its general meaning in the art and refers to a cytokine which is an important mediator of allergic inflammation and disease. The term may include naturally occurring IL13 and variants and modified forms thereof. The IL13 can be from any source, but typically is a mammalian (e.g., human and non-human primate) IL13, particularly a human IL13. An exemplary human native IL13 amino acid sequence is provided in GenPept database under accession number NP_002179 and an exemplary human native nucleotide sequence encoding for IL13 is provided in GenBank database under accession number NM_002188.

The SNP rs1800925 is well known in the art and is notably described in the NCBI database dbSNP. It consists in a variation of a cytosine in a thymine and is located in the promoter region of the IL13 gene sequence, at the position -1069 according to the numbering system described in den Dunnen T. et al, 2001 (written -1069C>T in related nomenclature).

The term *"MLHI* or *"MutL homolog 1"* has its general meaning in the art and refers to the human homolog of the *E. coli* MutL gene, a key component of the DNA mismatch repair (MMR) system. The term may include naturally occurring MLH1 and variants and modified forms thereof. The MLHI can be from any source, but typically is a mammalian (e.g., human and non-human primate) MLHl, particularly a human MLHl. An exemplary human native MLHI amino acid sequence is provided in GenPept database under accession number NP_000240 and an exemplary human native nucleotide sequence encoding for MLH1 is provided in GenBank database under accession number NM_000249.

The SNP rs1800734 is well known in the art and is notably described in the NCBI database dbSNP. It consists in a variation of a guanine in a adenine and is located in the promoter region of the MLHI gene sequence, at the position -93 according to the numbering system described in den Dunnen T. et al, 2001 (written -93G>A in related nomenclature).

In the context of the invention, the term *"patient"* refers to any subject, preferably a mammal, and more preferably a human, afflicted with an epithelial cancer likely to benefit from a chemoradiation treatment, in particular a rectal cancer.

According to the invention, the term "*biological sample*" refers to any sample isolated from a patient which permits DNA extraction. It can include, by way of example and not limitation, bodily fluids and/or tissue extracts such as homogenates or solubilised tissue obtained from a patient. Tissue extracts are obtained routinely from tissue biopsy and autopsy material. Bodily fluids useful in the present invention include blood, urine, saliva or any other bodily secretion or derivative thereof. As used herein "blood" includes whole blood, plasma, serum, circulating epithelial cells, constituents, or any derivative of blood. In a preferred embodiment of the invention, the sample to be tested is blood.

The terms *"treating"* or *"treatment"* refer to reversing, alleviating, inhibiting the progress of the disorder or condition to which such term applies, or one or more symptoms of such disorder or condition.

The term *"epithelial cancer"* refers to several cancers of epithelial tissues. Such cancers can be, but are not limited to, rectal, breast, prostate, lung and ENT (ear, nose, and throat) cancers.

The term *"responsiveness of a patient", "responder patient"* or *"responsive patient"* refers to a patient who shows or will show a clinically significant relief in the disease (e.g. cancer) during and/or after treatment. The disease activity can be measured according to standards recognized in the art.

According to the invention, the evaluation of the responsiveness to treatment can be evaluated by several methods well known in the art. For example, histopathologic examination of the surgically resected rectal carcinoma according to the tumor regression grade (TRG) system proposed by Dworak O. et al., 1997 can be used. Typically, in the example, patients with TGR 0, 1 or 2 are defined as non-responders patients and those with TGR 3 or 4 can be classified as responders.

Other evaluation methods can also be used such as the system proposed in Wheeler JMD et al, 2002 based on a 3 classes staging system. Finally downstaging or downsizing based on CT (computed tomography), ERUS (endorectal ultrasonography) or MR (magnetic resonance) imaging may also be used to evaluate treatment response (Gosens MJ, et al, 2007; Delaini GG, 2005)

### Methods of the invention

The method of the invention is based on the identification of particular genotypes which allow distinguishing patients between responders and non-responders to a chemoradiation treatment.

A first object of the invention relates to *a*n *in vitro* method for predicting the responsiveness of a patient afflicted with an epithelial cancer to a chemoradiation treatment, said method comprising the step of determining in a biological sample obtained from said patient the genotypes of the following single nucleotide polymorphism (SNP):
- rs744154 G>C in the ERCC4 gene,
- rs4880 T>C in the SOD2 gene,
- rs1800925 C>T in the IL13 gene, or
- rs1800734 G>A in the MLHI gene.

In one embodiment, the invention relates to the *in vitro* method of the invention, wherein the presence of the following genotypes:
- G/G for the SNP rs744154 G>C and C/C for the SNP rs4880 T>C; or
- G/G for the SNP rs744154 G>C and C/C for the SNP rs1800925 C>T; or
- G/G for the SNP rs744154 G>C and G/G for the SNP rs1800734 G>A; or
- G/G for the SNP rs744154 G>C, C/C for the SNP rs4880 T>C, C/C for the SNP rs1800925 C>T and G/G for the SNP rs1800734 G>A; or
- G/G for the SNP rs744154 G>C, C/C for the SNP rs4880 T>C and C/C for the SNP rs 1800925 C>T; or
- G/G for the SNP rs74154 G>C, C/C for the SNP rs4880 T>C and G/G for the SNP rs 1800734 G>A; or
- G/G for the rs744154 SNP G>C, C/C for the SNP rs1800925 C>T and G/G for the SNP rs 1800734 G>A; or
- C/C for the SNP rs1800925 C>T, G/G for the SNP rs1800734 G>A and C/C for the SNP rs4880 T>C
is indicative of a likelihood of at least 75% of responsiveness of said patient to a chemoradiation treatment.

In a particular embodiment, the presence of the following genotypes:
- G/G for the SNP rs744154 G>C, C/C for the SNP rs4880 T>C, C/C for the SNP rs1800925 C>T and G/G for the SNP rs1800734 G>A; or
- G/G for the SNP rs744154 G>C, C/C for the SNP rs4880 T>C and C/C for the SNP rs 1800925 C>T; or
- G/G for the SNP rs74154 G>C, C/C for the SNP rs4880 T>C and G/G for the SNP rs 1800734 G>A; or
- G/G for the rs744154 SNP G>C, C/C for the SNP rs1800925 C>T and G/G for the SNP rs1800734 G>A; or
- C/C for the SNP rs1800925 C>T, G/G for the SNP rs1800734 G>A and C/C for the SNP rs4880 T>C
is indicative of a likelihood of at least 90% of responsiveness of said patient to a chemoradiation treatment.

In another embodiment, the invention relates to *s*aid *in vitro* method wherein the presence of the following genotypes:
- C/C or C/G for the SNP rs744154 G>C and C/T or T/T for the SNP rs4880 T>C and C/T or T/T for the SNP rs1800925 C>T
is indicative of a likelihood of less than 20% of responsiveness of said patient to chemoradiation treatment.

Preferably, the genotype of a patient is determined on a nucleic acid sample obtained from a biological sample from said patient.

The nucleic acid sample may be obtained from any cell source or tissue biopsy. Nonlimiting examples of cell sources available include without limitation blood cells, buccal cells, epithelial cells, fibroblasts, or any cells present in a tissue obtained by biopsy. Cells may also be obtained from body fluids, such as blood or lymph, etc. DNA may be extracted using any methods known in the art, such as described in Sambrook J. et al., 1989.

The SNPs may be detected in the nucleic acid sample, preferably after amplification. For instance, the isolated DNA may be subjected to amplification by polymerase chain reaction (PCR), using oligonucleotide primers that are specific for one defined genotype or that enable amplification of a region containing the polymorphism of interest. According to a first alternative, conditions for primer annealing may be chosen to ensure specific reverse transcription (where appropriate) and amplification; so that the appearance of an amplification product be a diagnostic of the presence of a particular genotype. Otherwise, DNA may be amplified, after which the genotype is determined in the amplified sequence by hybridization with a suitable probe or by direct sequencing, or any other appropriate method known in the art.

As used herein, the terms *"primer"* (or *"amplification primer")* and *"probe"* refer to oligonucleotides which have different functions. A primer is typically extended by polymerase or ligation following hybridization to the target but a probe typically is not. A hybridized oligonucleotide may function as a probe if it is used to capture or detect a target sequence, and the same oligonucleotide may function as a primer when it is employed as a target binding sequence in an amplification primer.

Actually numerous strategies for genotype analysis are available. For example, RFLP (restriction fragment length polymorphism) may be used. Briefly, the nucleic acid molecule may be tested for the presence or absence of a restriction site. When a base substitution creates or abolishes the recognition site of a restriction enzyme, this allows a simple direct PCR test for the polymorphism. Further strategies include, but are not limited to, direct sequencing, hybridization with allele-specific oligonucleotides (ASO) that are short synthetic probes which hybridize only to a perfectly matched sequence under suitably stringent hybridization conditions; allele-specific PCR; PCR using mutagenic primers; ligase-PCR, real-time quantitative PCR, or oligonucleotide ligation assay (OLA). OLA may be used for revealing polymorphisms. According to this method, two oligonucleotides are constructed that hybridize to adjacent sequences in the target nucleic acid, with the join sited at the position of the mutation. DNA ligase will covalently join the two oligonucleotides only if they are perfectly hybridized. Direct sequencing may be accomplished by any method, including without limitation enzymatic sequencing, using the Sanger method; mass spectrometry sequencing; sequencing using a chip-based technology. Alternatively, scanning methods can be employed followed by one approach allowing the exact identification of base modification, as for example HOT cleavage; denaturing gradient gel electrophoresis (DGGE), temperature denaturing gradient gel electrophoresis (TGGE), single-stranded conformational polymorphism (SSCP) and denaturing high performance liquid chromatography, high resolution melting (HRM).
Preferably, DNA from the patient is first subjected to amplification by polymerase chain reaction (PCR) using specific amplification primers. However several other methods are available, allowing DNA to be studied independently of PCR, such as the rolling circle amplification (RCA), the InvaderTMassay. Moreover other amplification strategies allowing DNA amplification and PCR-free may also be suitable such as 3SR (Fahy E et al, 1991).

The SNPs of the invention may be identified by using DNA chip technologies as those described in documents WO 2004106546 and WO 2006001627.

Nucleic acids having at least 10 nucleotides and exhibiting sequence complementarity or homology to the sequence of interest herein find utility as hybridization probes or amplification primers. It is understood that such nucleic acids need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, and enzymatic or other ligands (e. g. avidin/biotin). Probes typically comprise single-stranded nucleic acids of between 10 to 1000 nucleotides in length, for instance of between 10 and 800, more preferably of between 15 and 700, typically of between 20 and 500.

Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaC1, 0.015 M Na-citrate).

According to another aspect of the invention, the SNPs of interest are detected by contacting the nucleic sample of the patient with a nucleic acid probe, which is optionally labelled. Primers may also be useful to amplify or sequence the portion of genes of the invention, e.g. ERCC4, SOD2, IL-13 and MLH1, containing the SNPs of interest.

In one embodiment, methods of the invention further comprise determining the genotypes of SNPs in linkage disequilibrium with the SNPs of the invention.

The term "linkage disequilibrium" (LD) refers to a population association among alleles at two or more loci. It is a measure of co-segregation of alleles in a population. Linkage disequilibrium or allelic association is the preferential association of a particular allele or genetic marker with a specific allele, or genetic marker at a nearby chromosomal location more frequently than expected by chance for any particular allele frequency in the population.

SNPs in linkage disequilibrium are equivalent to SNPs of the invention and thus provide an equivalent predictive value. Test for SNPs in linkage disequilibrium with the four SNPs of the invention can be performed by statistical techniques well known in the art. For example, the linkage disequilibrium could be evaluated by using the Lewontin's coefficient D' and the correlation coefficient r².

The chemoradiation treatment (also called "radiochemotherapy") refers to a treatment based on the association of concurrent radiotherapy and chemotherapy, classically used for treating cancers. The radiotherapy and the chemotherapy can vary and have to be adapted for such association.

The classic protocols of radiotherapy can be employed. For example, the total dose can be of 45 or 50 Gy, and can be delivered in 25 fractions of 1.8 to 2 Gy. But radiotherapy doses vary according to several parameters. Thus, different radiotherapy treatment protocols and doses can be used according to the invention.

The concurrent chemotherapy can for example consist in capecitabine used alone or in combination with oxaliplatin. 5-FU and analogues can also be used. Such as radiotherapy protocols, chemotherapy protocols vary according several parameters. Thus, different chemotherapy treatment protocols and doses can be used according to the invention.

In a particular embodiment of the invention, said epithelial cancer is a rectal cancer.

In a particular embodiment of the invention, the chemoradiation treatment of the *in vitro* method of the invention is a preoperative treatment.

### Kits of the invention

A second object of the invention refers to a kit for predicting the responsiveness of a patient afflicted with an epithelial cancer to a chemoradiation treatment, comprising:
- at least one primer and/or at least one probe for amplification or detection of a sequence comprising a SNP selected from the group consisting of rs744154 G>C, rs4880 T>C, rs1800925 C>T and rs1800734 G>A,
- hybridization reagents, and
- instructions for use.

In one embodiment of the invention, the primer or probe may be labelled with a suitable marker. Alternatively, the primer or probe may be unlabelled and the ingredients for labelling may be included in the kit in separate containers.

In another embodiment of the invention, the primer or probe may be coated on an array.

In a particular embodiment, the kit of the invention comprises a pair of nucleotides primers or a probe specific for amplifying or detecting each of the four SNPs of the invention, namely rs744154 G>C, rs4880 T>C, rs1800925 C>T and rs1800734 G>A.

The invention will be further illustrated by the following figures and examples. However, these examples and figures should not be interpreted in any way as limiting the scope of the present invention.

### FIGURES

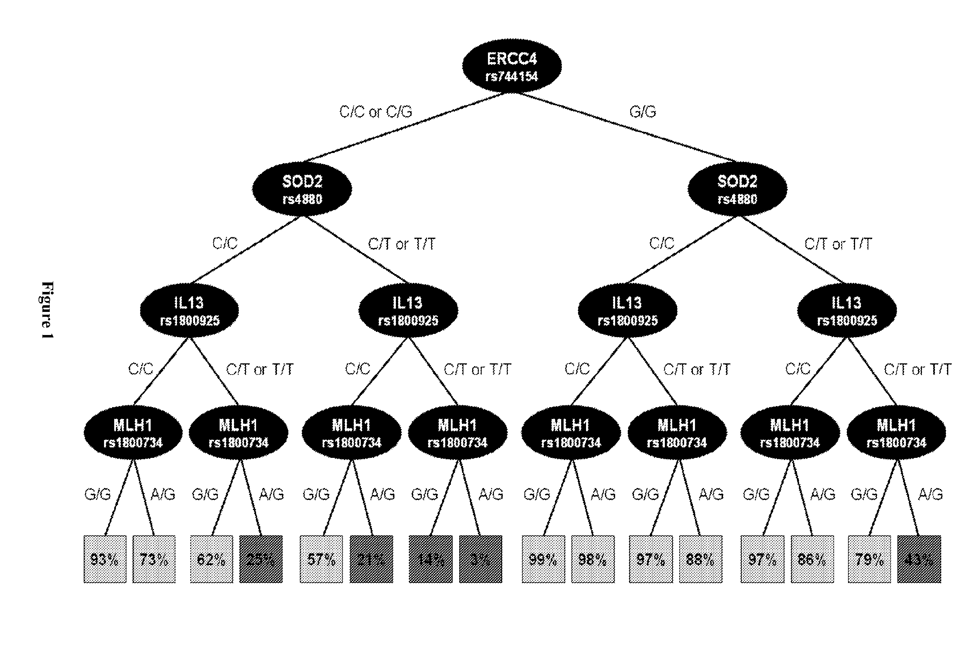

**Figure 1****.** Decision tree predicting the probability of response to a radiochemotherapy (RCT) for all possible multi-SNP combinations. Squares indicate the probability of response for each combination. Light and dark grey squares correspond to subgroups with high (>50%) or low chance (<50%) of response, respectively.

### EXAMPLE

### Material & Methods

### Patients

Between November 2005 and May 2008, 71 Caucasian patients with histologically confirmed rectal adenocarcinoma were recruited in a prospective study conducted at the Val d'Aurelle Paul-Lamarque Cancer Center in Montpellier. All patients underwent preoperative radiotherapy (total dose of 45 or 50 Gy, delivered in 25 fractions of 1.8 or 2 Gy over five weeks) with concurrent chemotherapy (capecitabine 1600 mg/m² daily or capecitabine + oxaliplatin 50 mg/m² weekly). Surgery was scheduled to be performed 6-8 weeks after the completion of preoperative radiochemotherapy (RCT). For all patients, informed consent for the use of clinical records and blood sample for research purposes was obtained. The protocol was approved by the Comité de Protection des Personnes of Saint-Eloi Hospital (Montpellier, France), a French Ethic committee for the protection of patients involved in biomedical research.

### Response Evaluation

Tumor response to preoperative RCT was evaluated by histopathologic examination of the surgically resected rectal carcinoma, according to the tumor regression grade (TRG) system proposed by Dworak O. et al., 1997. The entire primary tumor was paraffin-embedded, and tumor regression was semiquantitatively determined by the amount of residual carcinoma cells vs. the amount of fibrosis or mucin pools. The TRG ranges from TRG 0 when no fibrosis is visible (no regression), to TRG 4 when no viable tumor cells are detected (complete response). Tumor regression grade 1 = dominant tumor mass and obvious fibrosis or mucin; TRG 2 = dominantly fibrotic or mucinous changes, with few tumor cells or groups; and TRG 3 = very few tumor cells in fibrotic or mucinous tissue. Patients with TRG 0, 1 or 2 were defined as nonresponders, whereas those with TRG 3 or 4 were classified as responders.

### Selection of Genes and Polymorphisms

Based upon analysis of the SNP500Cancer database (Packer B.R. et al., 2006) and review of the literature, a total of 128 SNPs distributed over 76 genes were chosen for the analysis (Table 1). Selected SNPs had > 5% minor allele frequency according to the NCBI dbSNP database. Most of the candidate genes were reported to be related to cancer risk or outcome, but, a few other potential candidate genes or newly described SNPs were also included in our panel.

**Table 1. List of the Studied Candidate Genes and SNPs. *: rs number not available, SNPs are defined according to the nomenclature described in Den Dunnen J.T. et al. 2001.**

| **Gene** | **SNP** |
|---|---|
| | **DNA repair** |
| ADPRT | rs1136410 |
| APE1 | rs1130409, rs1760944 |
| ATM | rs189037, rs1801516, rs1800057 |
| BRCAI | rs1799966, rs16941, rs16942, rs799917 |
| BRCA2 | rs1799943, rs206143, rs144848 |
| CHEKl | rs521102 |
| CHEK2 | rs2267130 |
| ERCC1 | rs4803823, rs11615 |
| ERCC2 | rs28365048 |
| ERCC4 | rs1799802, rs744154, rs1799801, rs1799800 |
| ERCC5 | rs17655 |
| LIG3 | rs1052536, rs3135967 |
| LIG4 | rs1805388, rs1805386 |
| MBD4 | rs10342, rs140693 |
| MGMT | rs12917 |
| MLHI | rs1799977, rs1800734 |
| NBN | rs1805794 |
| OGGl | rs1052133 |
| RAD 51 | rs5030783, rs1801321 |
| RAD52 | rs11226 |
| RECQL | rs13035 |
| TP53BPI | rs2602141, rs560191 |
| XPA | rs3176658 |
| XRCC1 | rs25487, rs25489, rs1799782, rs3213245 |
| XRCC 3 | rs861539, rs1799794 |
| XRCC5 | rs1051677, rs1051685, rs6941, rs2440 |
| ZNF350 | rs2278415, rs2278420 |

| | **Apoptosis** |
|---|---|
| BAX | rs36017265, rs4645878 |
| BCL2 | rs2279115 |
| CASP3 | rs6948, rs1049216 |
| CASP8 | rs1045485, rs13113 |
| CASP9 | rs1052576 |
| CASP10 | rs13010627 |

| | **Cell cycle** |
|---|---|
| CCND1 | s603965 |
| CDKN1A | rs1801270 |
| MDM2 | rs1470383 |
| TP53 | rs1042522 |
| TP73 | rs2273953, rs1801173 |

| | **Growth factors / growth factor receptors** |
|---|---|
| EGF | rs4444903 |
| EGFR | rs11543848, rs712829, rs712830, rs17335738, rs17290169 |
| ERBB2 | rs1801200 |
| FGF2 | rs308447 |
| FGFR4 | rs351855 |
| IGF1R | rs2229765 |
| IGF2R | rs629849 |
| TGFBI | rs1982073, rs1800471, rs1800469 |
| VEGFA | rs2010963, rs1570360, rs699947, rs3025039 |

| | **Immune response** |
|---|---|
| FCGR2A | rs1801274 |
| FCGR3A | rs396991 |
| IL1B | rs1143627, rs1143634 |
| IL4 | rs2243250 |
| IL6 | rs1800795 |
| IL8 | rs4073 |
| IL10 | rs1800896 |
| IL13 | rs20541, rs1800925 |
| LTA | rs2229094 |
| NFKB1 | rs3774932, rs3774934, rs3774936, rs3774937 |
| PTGS2 | rs20417 |
| PPARG | rs1801282 |
| TNF | rs1800629 |

| | **Transcription factors** |
|---|---|
| NFE2L2 | -686A>G*, -650C>A*, rs5031039 |
| PARP-1 | rs1136410 |

| | **Beta-catenin pathway** |
|---|---|
| CTNNB1 | rs13072632, rs4135385 |
| GSK3B | rs3755557, rs334558 |

| | **Oxidative stress** |
|---|---|
| GPX1 | rs1050450 |
| MPO | rs7208693 |
| NOS2A | rs2297518 |
| NOS3 | rs1799983 |
| SOD2 | rs4880 |

| | **Adhesion** |
|---|---|
| ICAMS | rs1056538, rs2228615 |

| | **Hypoxia** |
|---|---|
| HIF1A | rs11549465, rs11549467, rs2246350 |

| | **Metabolism** |
|---|---|
| CYP1A1 | rs1048943 |
| GSTP1 | rs1695 |
| GSTT1 | rs4630 |
| MTHFR | rs1801133, rs1801131 |
| MT-ND3 | rs2853826 |

### DNA Extraction and SNP Genotyping

For all patients, whole blood was collected at the time of enrollment, and genomic DNA was extracted from peripheral lymphocytes using the QIAmp DNA blood maxi kit (Qiagen). The majority of the SNPs were genotyped by IntegraGen (Evry, France) with the use of an oligonucleotide ligation assay (SNPlex™) or an allelic discrimination assay (TaqMan^{®}). Six SNPs (rs603965, rs2227983, rs712829, rs712830, rs17335738 and rs17290169) were characterized in our laboratory by PCR-RFLP or direct sequencing (protocol available on demand).

### Statistical Analyses

All statistical analyses were performed with the STATA 10.0 (StataCorp, College Station, TX) and R, Version 2.8.1 (R Foundation for Statistical Computing) softwares. For each SNP, deviation from the Hardy-Weinberg equilibrium (HWE) was tested using the R package SNPStats (Solé X. et al., 2006). Those that were not in agreement with HWE were excluded from the analysis. Linkage disequilibrium (LD) was evaluated using the Lewontin's s coefficient D' and the correlation coefficient *r².* The association between individual SNPs and response to preoperative RCT was tested by logistic regression using the R package SNPassoc (González J.R. et al., 2007). For each SNP, genotypes were analyzed as a three-group categorical variable (co-dominant model), and they were also grouped according to the dominant (both the heterozygous variant and the rare homozygous variant were combined) and recessive (both the heterozygous variant and the homozygous variant with the most frequent allele were combined) model. The best model was chosen based on Akaike Information Criteria. For the clinicopathologic features, univariate analyses comparing responder and non-responder patients were performed by Pearson's chi-squared or Fisher's exact test for categorical variables and by the two-sample Wilcoxon test for all continuous variables. Differences were considered statistically significant when p < .05. In the multivariate analyse, stepwise logistic regression was performed to identify independent predictors of response and calculate the probability of response (Pr) for each combination.

### Results

### Pathological Responses to CRT

Thirty-two (45.1%) of 71 patients showed a good response to preoperative RCT, with 9 patients displaying pathological complete regression (TRG 4) and 23 presenting only microscopic residual tumors (TRG 3). Thirty-nine (54.9%) patients were considered non-responders, with 2, 16 and 21 patients evaluated as TRG 0, TRG 1 and TRG 2, respectively.

### Genotypes, HWE, and LD

Twenty of the SNPs (rs2246350, rs308447, rs2297518, rs699947, rs1982073, rs1800469, rs1801282, rs6941, rs1799802, rs1760944, rs1136410, rs140693, rs10342, rs3176658, rs4630, rs1048943, rs2267130, rs1805386, rs17290169 and NFE2L2 -650C>A) did not conform to Hardy-Weinberg equilibrium (P ≤ .05) and three (rs5031039, rs1 154946 rs36017265) were monomorphic. Strong linkage disequilibrium (LD) could be demonstrated for the following SNPs: TP73 rs2273953 and rs1801173 (D' = 1, *r²* = 1), ICAM5 rs1056538 and rs2228615 (D = 1, *r²*= 1), NFKBI rs3774937 and rs3774936 (D' = 1, *r²* = 1), XRCC5 rs1051677 and rs6941 (D' = 0.99, *r²* = 0.98), and TP53BPI rs2602141 and rs560191 (D' = 0.99, *r²* = 0.96). In addition, BRCAI rs16941 was in strong LD with rs16942 (D' = 1, ,r2 = 1), rs1799966 (D' = 1, *r²* = 1) and rs799917 (D' = 0.99, *r²* = 0.90), and ERCC4 rs744154 was in strong LD with rs1799800 (D' = 1, *r²* = 1) and rs1799801 (D' = 0.96 , *r²* = 0.92). Therefore, among linked SNPs, association analyses considered the SNP with the highest number of successful genotypes. Ninety-three (98 %) of the remaining 95 SNPs showed minor allele frequencies of > 5 %.

### Univariate analysis

SNPs were individually tested for association with response to preoperative RCT by logistic regression using different genetic models (co-dominant, dominant, recessive). The polymorphic genotypes of CASP10 rs13010627, SOD2 rs4880, IL13 rs1800925, MLHI rs1800734, and ERCC4 rs744154 were significantly different between patients who responded and those who did not respond to the neoadjuvant treatment (Table 2). For all four SNPs, the calculated allele frequency distributions were similar to the mean prevalences described in the NCBI SNP database for the Caucasian ethnicity. No statistical significance was found between the pathologic response and pretherapeutic clinicopathologic parameters, total irradiation dose or chemotherapy regimen. As expected, the pathologic T stage was significantly correlated with the response. Tumors that responded well to preoperative RCT according to the TRG were associated with a lower pathologic T stage.

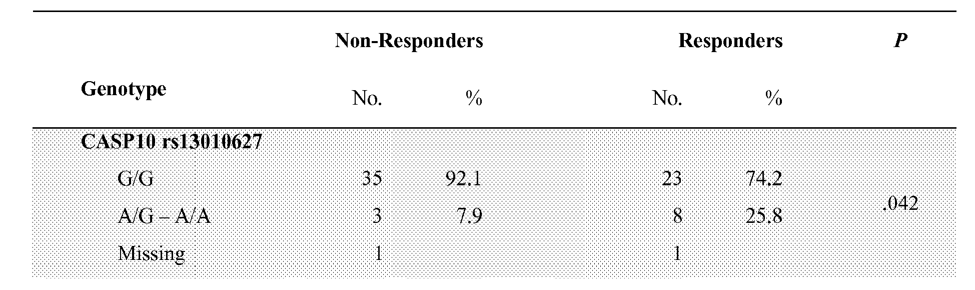

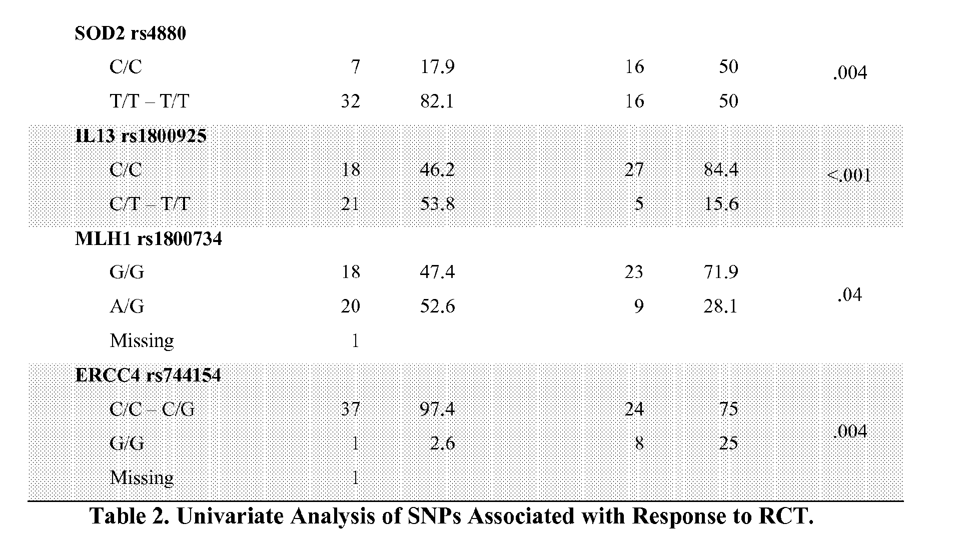

### Multivariable analysis

A multivariate logistic regression analysis indicated that SOD2 rs4880, IL13 rs1800925, MLH1 rs1800734, and ERCC4 rs744154 were the only markers demonstrating independent predictive value for pathologic response after adjustment for other SNPs (Table 3). ERCC4 rs744154 G/G carriers were more likely to respond to preoperative RCT that C/C and C/G carriers (OR, 23.24; 95% CI, 1.53 to 353.7; P = .008). On the contrary, patients with the SOD2 rs4880 C/T or T/T genotypes had a significantly lower chance of response to preoperative RCT than patients bearing the C/C genotype (OR, 0.10; 95% CI, 0.02 to 0.47; P = .001). Similarly, patients with the IL13 rs1800925 C/T and T/T genotypes and those with the MLHl rs1800734 A/G genotype had a significantly lower chance of response than those harbouring the IL13 rs1800925 C/C genotype (OR, 0.12; 95% CI, 0.03 to 0.49; P = .001) and those having the MLH1 rs1800734 G/G genotype (OR, 0.20; 95% CI, 0.05 to 0.77; P = .012), respectively.

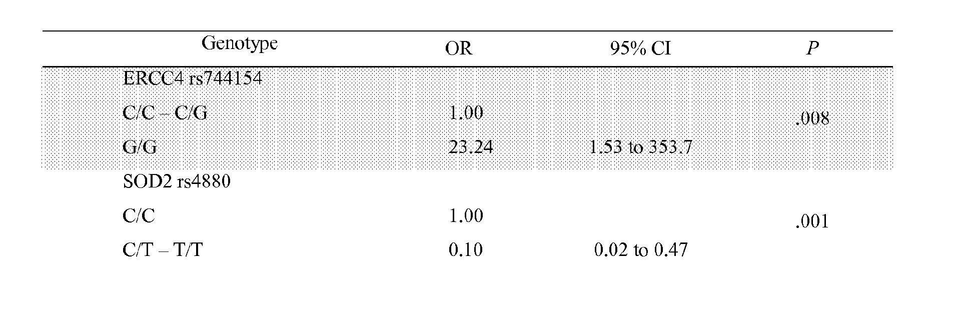

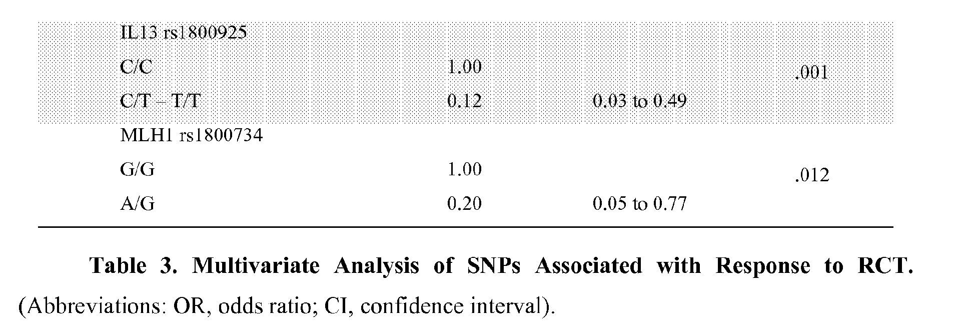

To assess the ability of these genetic markers to be used in a clinical setting, we developed a decision tree where the expected probability of response (Pr) for each SNPs combination is calculated (Figure 1). Patients with > 50% chance of response (Pr > 0.5) were predicted to be responders. The four-SNP classifier correctly predicted the response of 80% (95% CI, 69% to 89%) of patients. The area under the receiver-operating-characteristic (ROC) curve associated to our model was 0.87 (95% CI, 0.77 to 0.94), which indicates high discrimination between responders and non-responders. The sensitivity of the predictive model (percentage of responders who were predicted correctly as responders) was 81% (95% CI, 64% to 93%).The specificity (percentage of non-responders who were predicted correctly as non-responders) was 79% (95% CI, 63% to 90%). The positive predictive value (percentage of patients classified as responders who were true responders) was 76% (95% CI, 59% to 89%), and the negative predictive value (percentage of patients classified as non-responders who were true non-responders) was 83% (95% CI, 67% to 94%).

### REFERENCES

Throughout this application, various references describe the state of the art to which this invention pertains. The disclosures of these references are hereby incorporated by reference into the present disclosure.
Den Dunnen JT, Antonarakis SE. Hum Genet 109(1):121-124, 2001.
Dworak O, Keilholz L, Hoffmann A: Pathological features of rectal cancer after preoperative radiochemotherapy. Int J Colorectal Dis. 12:19-23, 1997.
Fahy E, Kwoh DY, Gingeras TR. Self-sustained sequence replication (3SR): an isothermal transcription-based amplification system alternative to PCR. PCR Methods Appl. 1991 Aug; 1(1):25-33
Gosens MJ, Klaassen RA, Tan-Go I, Rutten HJ, Martijn H, van den Brule AJ, Nieuwenhuijzen GA, van Krieken JH, Nagtegaal ID. Circumferential margin involvement is the crucial prognostic factor after multimodality treatment in patients with locally advanced rectal carcinoma. Clin Cancer Res. 2007 Nov 15; 13(22 Pt 1):6617-23 and G.G. Delaini Ed. Rectal Cancer: New frontiers in diagnosis, treatment and rheabilitation. Springer. 2005
González JR, Armengol L, Solé X, et al: SNPassoc: an R package to perform whole genome association studies. Bioinformatics 23:644-645, 2007
Grube D: Constants and variables in immunohistochemistry. Arch Histol Cytol 67:115-134, 2004
Kuremsky JG, Tepper JE, McLeod HL: Biomarkers for response to neoadjuvant chemoradiation for rectal cancer. Int J Radiat Oncol Biol Phys 74:673-688, 2009.
Packer BR, Yeager M, Burdett L, et al: SNP500Cancer: a public resource for sequence validation, assay development, and frequency analysis for genetic variation in candidate genes. Nucleic Acids Res 34:D617-621, 2006
Robert J, Morvan VL, Smith D, et al: Predicting drug response and toxicity based on gene polymorphisms. Crit Rev Oncol Hematol 54:171-196, 2005
Sambrook J, Fritsch EF, Maniatis T. Molecular cloning : a laboratory manual, 2nd edition. Cold Spring Harbor Laboratory Press, Cold Spring Harbor, New York. 1989.
Smith FM, Reynolds JV, Miller N, et al: Pathological and molecular predictors of the response of rectal cancer to neoadjuvant radiochemotherapy. Eur J Surg Oncol 32:55-64, 2006.
Solé X, Guinó E, Valls J, et al: SNPStats: a web tool for the analysis of association studies. Bioinformatics 22:1928-1929, 2006.
Spindler KG, Nielsen JN, Lindebjerg J, et al: Germline polymorphisms may act as predictors of response to preoperative chemoradiation in locally advanced T3 rectal tumors. Dis Colon Rectum 50:1363-1369, 2007
The International HapMap Consortium: The International HapMap Project. Nature 426:789-796, 2003
Ulrich CM, Robien K, McLeod HL: Cancer pharmacogenetics: polymorphisms, pathways and beyond. Nat Rev Cancer 3:912-920, 2003
Wheeler JMD, Warren BF, Mortensen NJM, Ekanyaka N, Kulacoglu H, Jones AC, George BD, and Kettlewell MGW. 2002. Quantification of histologic regression of rectal cancer after irradiation: a proposal for a modified staging system. Dis. Colon Rectum 45:1051-1056

## Claims

1. An *in vitro* method for predicting the responsiveness of a patient afflicted with a rectal cancer to a chemoradiation treatment, said method comprising the step of determining in a biological sample obtained from said patient the genotype of the following single nucleotide polymorphisms (SNP):
- rs744154 G>C in the ERCC4 gene,
- rs4880 T>C in the SOD2 gene,
- rs1800925 C>T in the IL-13 gene, or
- rs1800734 G>A in the MLH1 gene.

2. The *in vitro* method according to claim 1 wherein the presence of the following genotypes:
- G/G for the SNP rs744154 G>C and C/C for the SNP rs4880 T>C; or
- G/G for the SNP rs744154 G>C and C/C for the SNP rs1800925 C>T; or
- G/G for the SNP rs744154 G>C and G/G for the SNP rs1800734 G>A; or
- G/G for the SNP rs744154 G>C, C/C for the SNP rs4880 T>C, C/C for the SNP rs1800925 C>T and G/G for the SNP rs1800734 G>A; or
- G/G for the SNP rs744154 G>C, C/C for the SNP rs4880 T>C and C/C for the SNP rs 1800925 C>T; or
- G/G for the SNP rs74154 G>C, C/C for the SNP rs4880 T>C and G/G for the SNP rs 1800734 G>A; or
- a G/G for the rs744154 SNP G>C, C/C for the SNP rs1800925 C>T and G/G for the SNP rs 1800734 G>A; or
- C/C for the SNP rs1800925 C>T, G/G for the SNP rs1800734 G>A and C/C for the SNP rs4880 T>C
is indicative of a likelihood of at least 75% of responsiveness of said patient to a chemoradiation treatment.

3. The *in vitro* method according to any one of claims 1-2 wherein the presence of:
- G/G for the SNP rs744154 G>C, C/C for the SNP rs4880 T>C, C/C for the SNP rs1800925 C>T and G/G for the SNP rs1800734 G>A; or
- G/G for the SNP rs744154 G>C, C/C for the SNP rs4880 T>C and C/C for the SNP rs 1800925 C>T; or
- G/G for the SNP rs74154 G>C, C/C for the SNP rs4880 T>C and G/G for the SNP rs 1800734 G>A; or
- G/G for the rs744154 SNP G>C, C/C for the SNP rs1800925 C>T and G/G for the SNP rs 1800734 G>A; or
- C/C for the SNP rs1800925 C>T, G/G for the SNP rs1800734 G>A and C/C for the SNP rs4880 T>C
is indicative of a likelihood of at least 90% of responsiveness of said patient to a chemoradiation treatment.

4. The *in vitro* method according to any one of claims 1-3 wherein the chemoradiation treatment is a preoperative treatment.
